# EUROPEAN PATENT SPECIFICATION

(11) **EP 2 477 503 B1**
(45) Date of publication and mention of the grant of the patent: **31.07.2013**
(21) Application number: 10782533.3
(22) Date of filing: 16.09.2010
(51) Int. Cl.: A23C 9/13

(54) **PROCESS FOR PREPARING A PASTEURISED AND FERMENTED DAIRY PRODUCT SUPPLEMENTED WITH CALCIUM AND VITAMIN D**
HERSTELLUNG EINES MIT CALCIUM UND VITAMIN D ANGEREICHERTEN PASTEURISIERTEN UND FERMENTIERTEN MILCHPRODUKTS
PROCEDE POUR PREPARER UN PRODUIT LAITIER PASTEURISE ET FERMENTE SUPPLEMENTE AVEC DE LA CALCIUM ET DE LA VITAMIN D

(30) Priority: 17.09.2009 ES 200901332 U
(43) Date of publication of application: 25.07.2012
(73) Proprietor: Danone, S.A., E-08029 Barcelona (ES)
(72) Inventor: TRICOIRE, Philippe, E-08029 Barcelona (ES); ÁLVAREZ FERNÁNDEZ, Cristina, E-08029 Barcelona (ES); MONTSERRAT CARRERAS, Agustin, E-08029 Barcelona (ES); SANCHO DOMENC, Jordi, E-08029 Barcelona (ES)
(74) Representative: Linage González, Rafael
(86) International application number: PCT/EP2010/005702
(87) International publication number: WO 2011/066880

(56) References cited:
- EP-A1- 0 709 033
- EP-A1- 1 151 675
- WO-A1-02/43503
- WO-A1-2006/087409
- WO-A2-01/15715
- ES-A1- 2 036 481
- JACKSON REBECCA D ET AL: "Calcium plus vitamin D supplementation and the risk of fractures.", THE NEW ENGLAND JOURNAL OF MEDICINE 16 FEB 2006 LNKD- PUBMED:16481635, vol. 354, no. 7, 16 February 2006 (2006-02-16), pages 669-683, XP002623192, ISSN: 1533-4406
- DATABASE GNPD [Online] Mintel; April 2008 (2008-04), Anonymous: "Apricot Flavoured Yogurt", XP002623193, retrieved from www.gnpd.com Database accession no. 896978
- DATABASE GNPD [Online] Mintel; November 2007 (2007-11), Anonymous: "Low Fat Natural Yoghurt", XP002623194, retrieved from www.gnpd.com Database accession no. 807717
- DATABASE GNDP [Online] Mintel; March 2008 (2008-03), Anonymous: "Low Fat Strawberry Yoghurt", XP002642271, retrieved from www.gnpd.com Database accession no. 880102
- WHITE K M ET AL: "Changes in body composition with yogurt consumption during resistance training in women.", INTERNATIONAL JOURNAL OF SPORT NUTRITION AND EXERCISE METABOLISM 2009 DIV. OF HEALTH SCI.,, vol. 19, no. 1, February 2009 (2009-02), pages 18-33, XP002623195,
- CHAIWANON P ET AL: "CALCIUM FORTIFICATION IN SOYBEAN MILK AND IN VITRO BIOAVAILABILITY", JOURNAL OF FOOD COMPOSITION AND ANALYSIS, ACADEMIC PRESS, LONDON, GB, vol. 13, 1 January 2000 (2000-01-01), pages 319-327, XP001197306, ISSN: 0889-1575, DOI: DOI:10.1006/JFCA.1999.0854

## Description

### FIELD OF THE INVENTION

The present invention relates to a process for preparing a pasteurised and fermented dairy product with a new composition.

The field of the invention is therefore the dairy industry.

### BACKGROUND OF THE INVENTION

The preparation of dairy products enriched with a great diversity of vitamins and/or minerals is known. They all seek to provide the nutrients that are least present today in the diet of the general population, or those that are the most necessary for the food to provide the maximum health benefits possible. It is also known to pasteurise these products for reasons of health and durability.

Regarding enrichment, amongst the minerals, calcium is an important addition in human diets for the adequate health of bones and teeth, as well as for the correct development of certain body functions such as the transmission of nervous signals, blood coagulation, muscle contractions, etc. This makes the enrichment of food products with calcium a common practice.

However, if highly soluble calcium sources are used (calcium chloride, etc.), the calcium may interact with ingredients that are sensitive to calcium, such as milk proteins, which could lead to the coagulation of these ingredients during heat treatment, such as pasteurisation.

Calcium sources that are essentially insoluble are therefore used to enrich non-liquid products (i.e. with yogurt-like textures), sources such as calcium citrate, for example, together with a stabiliser. Calcium citrate has advantages with respect to other calcium salts when used in enriched foods, due to its high bioavailability. Moreover, calcium citrate, in contrast to calcium in general, only interferes marginally in the absorption of other minerals, especially iron. That is, a long term calcium supplement with calcium citrate may, for example, reduce the risk of formation of kidney and urinary stones, since the citrate ions inhibit the crystallisation of the calcium salts involved in the formation of stones.

It is also important to enrich calcium-enriched products with vitamin D, since this vitamin regulates the incorporation of calcium into the bones, and without it the bones would receive no benefits from the added calcium. Vitamin D also regulates phosphorous blood levels, regulates calcium reabsorption in the kidneys and participates in the inhibition of parathyroid hormone (PTH) secretion from the parathyroid gland, or influences the immune system due to its immunosuppressive action, promoting phagocytosis and antitumor activity.

Physicians usually recommend an intake of about 800 mg of calcium daily in order to maintain optimal blood levels for an average person (between 18-50 years of age). This dose is slightly lower for children and greater for teenagers and adults in specific situations (e.g., during pregnancy).

Similarly, physicians also recommend a daily intake of about 5 µg of vitamin D for an average person (up to 50 years of age), which increases with age.

Document JACKSON REBECCA D ET AL "Calcium plus vitamin D supplementation and the risk of fractures », THE NEW ENGLAND JOURNAL OF MEDECINE 16 FEB 2006 LNKD-PUBMED :1533-4406, vol. 354, no 7, 16 February 2006, pages 669-683, describes the efficacy of calcium with vitamin D supplementation for the prevention of fractures. Sujects are administered dairy products with low concentrations of calcium and/or vitamin D. There is a need for processes for preparing products with higher concentrations of both calcium and vitamin D.

Document GNPD [Online], Mintel, April 2008 "Apricot Flavored Yogurt*"* describes yogurt having concentrations, per 100 g, of 400 mg Calcium and 1.25 µg vitamin D. The production process is not described. There is a need for processes for preparing products with higher concentrations of both calcium and vitamin D.

Document GNPD [Online], Mintel, November 2007 "Low Fat Natural Yogurt*"* describes yogurt having concentrations, per 100 g, of 264 mg Calcium and 3.3 µg vitamin D. The production process is not described. There is a need for processes for preparing products with higher concentrations of both calcium and vitamin D.

Document WO 01/15715 describes a process for making yogurts by adding after fermentation calcium and vitamin D, to reach concentrations, per 100 g, of 200 mg Calcium and 3 µg vitamin D. There is a need for processes for preparing products with higher concentrations of both calcium and vitamin D.

Document GNPD [Online], Mintel, March 2008 "Low Fat Strawberry Yogurt*"* describes yogurt having concentrations, per 100 g, of 100 mg Calcium and 3 µg vitamin D. The production process is not described. There is a need for processes for preparing products with higher concentrations of both calcium and vitamin D.

Document WO 02/43503 describes a process for making yogurts by adding after fermentation calcium and vitamin D, to reach concentrations, per 100 g, of 250-764 mg Calcium and 1 µg vitamin D. There is a need for processes for preparing products with higher concentrations of both calcium and vitamin D.

### SUMMARY OF THE INVENTION

An object of the invention is therefore to provide a process for preparing a dairy product, specifically a pasteurised and fermented dairy product, which provides a high amount of calcium and a high amount of vitamin D. The product can typically provide at least 50% of the recommended dose of calcium mentioned above and at least 100% of the recommended dose of vitamin D mentioned above, per portion (125 g) of the product. That is, a pasteurised and fermented dairy product with the following amounts, for each 100 g of the product:
- at least 320 mg of calcium and at least 4 µg of vitamin D, or
- 150 mg ≤ calcium < 320 mg and 4 µg ≤ vitamin D < 10 µg.

### DETAILED DESCRIPTION OF AN EMBODIMENT OF THE INVENTION

In the present application, any amounts expressed in percentage refer to the percentage by weight of the total weight, unless stated otherwise.

In the present application, amounts of calcium are preferably provided as amounts at the expiry date of the product. The expiry date refers to a limit date provided on the packaging of the product. It might be a preferred date, such as "best before", or limit such as "consume before".

In the present specification "fat" in nutritional profiles refers indifferently to fat content or to lipid content.

In the present specification a value provided a nutritional profile covers the value itself ± 5% of said value, preferably the value itself ± 1%.

In the present specification the use of a product is intended to cover the use itself, optionally with the connected intention, but also to cover any communication with commercial or legal potential consequences, associated to the product, for example advertisement, instructions or recommendations on the package of product, instructions or recommendation on commercial support such as leaflets, brochures, posters, websites, documentation filed in support to regulatory registrations for safety purpose, efficacy purpose, or consumer protection, for example at administrations such as EFSA in Europe and FDA in USA.

The product of the process of the invention comprises the following amounts of calcium and vitamin D, for each 100 g of the product,
- at least 320 mg of calcium and at least 4 µg of vitamin D, or
- 150 mg ≤ calcium < 320 mg and 4 µg ≤ Vitamin D < 10 µg.

These amounts per 100 g of product can be also referred to as "concentrations".

In one embodiment the concentrations are as follows:
- calcium ≤ 1200 mg, preferably calcium ≤ 1000 mg, preferably calcium ≤ 750 mg, preferably calcium ≤ 600 mg, and
- vitamin D ≤ 10 µg, preferably vitamin D ≤ 8 µg, preferably vitamin D ≤ 7 µg, preferably vitamin D ≤ 6 µg.

In one embodiment the concentrations are as follows: calcium < 1000 mg and vitamin D < 8 µg.

In one embodiment the concentrations are as follows calcium < 750 mg and vitamin D < 7 µg.

In one embodiment the concentrations are as follows calcium < 600 mg and vitamin D < 6 µg.

In one embodiment the concentrations are as follows: 320 mg < calcium < 1000 mg and 4 µg < vitamin D < 10 mg, preferably 320 mg < calcium < 750 mg and 4 µg < vitamin D < 8 µg.

In one embodiment the concentrations are as follows: 150 mg ≤ calcium < 320 mg and 4 µg ≤ vitamin D < 10 µg, preferably 200 mg ≤ calcium < 320 mg and 4 µg ≤ vitamin D < 8 µg.

In one embodiment the concentrations are as follows: calcium = 320 mg and vitamin D = 4 µg. In one embodiment the concentrations are as follows: calcium > 320 mg and vitamin D = 4 µg. In one embodiment the concentrations are as follows: calcium = 320 mg and vitamin D > 4 µg. In one embodiment the concentrations are as follows: 320 mg < calcium and 4 µg < vitamin D.

In one embodiment the concentrations are as follows: calcium ≠ 320 mg and vitamin D ≠ 4 µg.

In one embodiment the product is different from a product contained is a container having a serving amount of 125 g, and concentrations of as follows: calcium = 320 mg and vitamin D = 4 µg.

In one embodiment the product is different from a product having the following nutritional profile:

| | Per 100 g | Per serving (125g) |
|---|---|---|
| Energy | 58 kcal | 73 kcal |
| Protein | 4.2 g | 5.2 g |
| Carbohydrate | 7 g | 8.7 g |
| Of which sugars (added sugar) | 6.2 g | 7.8 g |
| Fat | 1.5 g | 1.9 g |
| Of which saturates | 0.95 g | 1.2 g |
| Fibres | Trace or 0g | Trace or 0g |
| Sodium | 0.07 g | 0.09 g |
| Calcium | 320 mg | 400 mg |
| Vitamin D | 4 µg | 5 µg |

In one embodiment the product is different from a product having the following nutritional profile:

| | Per 100 g | Per serving (125g) |
|---|---|---|
| Energy | 70 kcal | 88 kcal |
| Protein | 4.2 g | 5.2 g |
| Carbohydrate | 9.8 g | 12.2 g |
| Of which sugars (added sugar) | 9.2 g | 11.5 g |
| Fat | 1.5 g | 1.9 g |
| Of which saturates | 0.95 g | 1.2 g |
| Fibres | Trace or 0g | Trace or 0g |
| Sodium | 0.07 g | 0.09 g |
| Calcium | 320 mg | 400 mg |
| Vitamin D | 4 µg | 5 µg |

In one embodiment the product is different from a product having the following nutritional profile:

| | Per 100 g | Per serving (125g) |
|---|---|---|
| Energy | 74 kcal | 92 kcal |
| Protein | 4.2 g | 5.2 g |
| Carbohydrate | 11 g | 13 g |
| Of which sugars (added sugar) | 10 g | 13 g |
| Fat | 1.6 g | 1.9 g |
| Of which saturates | 0.95 g | 1.2 g |
| Fibres | Trace or 0g | Trace or 0g |
| Sodium | 0.07 g | 0.09 g |
| Calcium | 320 mg | 400 mg |
| Vitamin D | 4 µg | 5 µg |

In one embodiment the product is different from a product having the following nutritional profile:

| | Per 100 g |
|---|---|
| Energy | 74 kcal |
| Protein | 4.7 g |
| Carbohydrate | 10.4 g |
| Of which sugars (added sugar) | 9.9 g |
| Fat | 1.5 g |
| Of which saturates | 1.0 g |
| Sodium | < 0.1 g |
| Calcium | 320 mg |
| Vitamin D | 4 µg |

In one embodiment the product is different from a product having the following nutritional profile:

| | Per 100 g |
|---|---|
| Energy | 89 kcal |
| Protein | 4.8 g |
| Carbohydrate | 14.1 g |
| Of which sugars (added sugar) | 13.7 g |
| Fat | 1.5 g |
| Of which saturates | 1.0 g |
| Sodium | < 0.1 g |
| Calcium | 320 mg |
| Vitamin D | 4 µg |

In one embodiment the product is different from a product having the following nutritional profile:

| | Per 100 g | Per 125 g |
|---|---|---|
| Energy | 87.2 kcal | 109 |
| Protein | 3.04 g | 3.8 |
| Carbohydrate | 13.04 g | 16.3 |
| Of which sugars (added sugar) | 12.72 g | 15.9 |
| Fat | 2.56 g | 3.2 |
| Of which saturates | 1.68 g | 2.1 |
| Fibers | 2.24 g | 0.3 |
| Sodium | 0.048 g | 0.06 |
| Calcium | 320 mg | 400 |
| Vitamin D | 4 µg | 5 |

In one embodiment the product is different from a product having the following nutritional profile:

| | Per 100 g |
|---|---|
| Energy | 89 kcal |
| Protein | 3.1 g |
| Carbohydrate | 13.6 g |
| Of which sugars (added sugar) | 13.2 g |
| Fat | 2.6 g |
| Of which saturates | 1.7 g |
| Fibers | 0.1 g |
| Sodium | 0,05 g |
| Calcium | 320 mg |
| Vitamin D | 4 µg |

In one embodiment the product is different from a product having the following nutritional profile:

| | Per 100 g |
|---|---|
| Energy | 75 kcal |
| Protein | 4.5 g |
| Carbohydrate | 9.9 g |
| Of which sugars (added sugar) | 9.1 g |
| Fat | 1.5 g |
| Of which saturates | 1 g |
| Fibers | 0,7 g |
| Sodium | 0,07 g |
| Calcium | 320 mg |
| Vitamin D | 4 µg |

In one embodiment the product is different from products having the following nutritional profiles per 100 g of product:

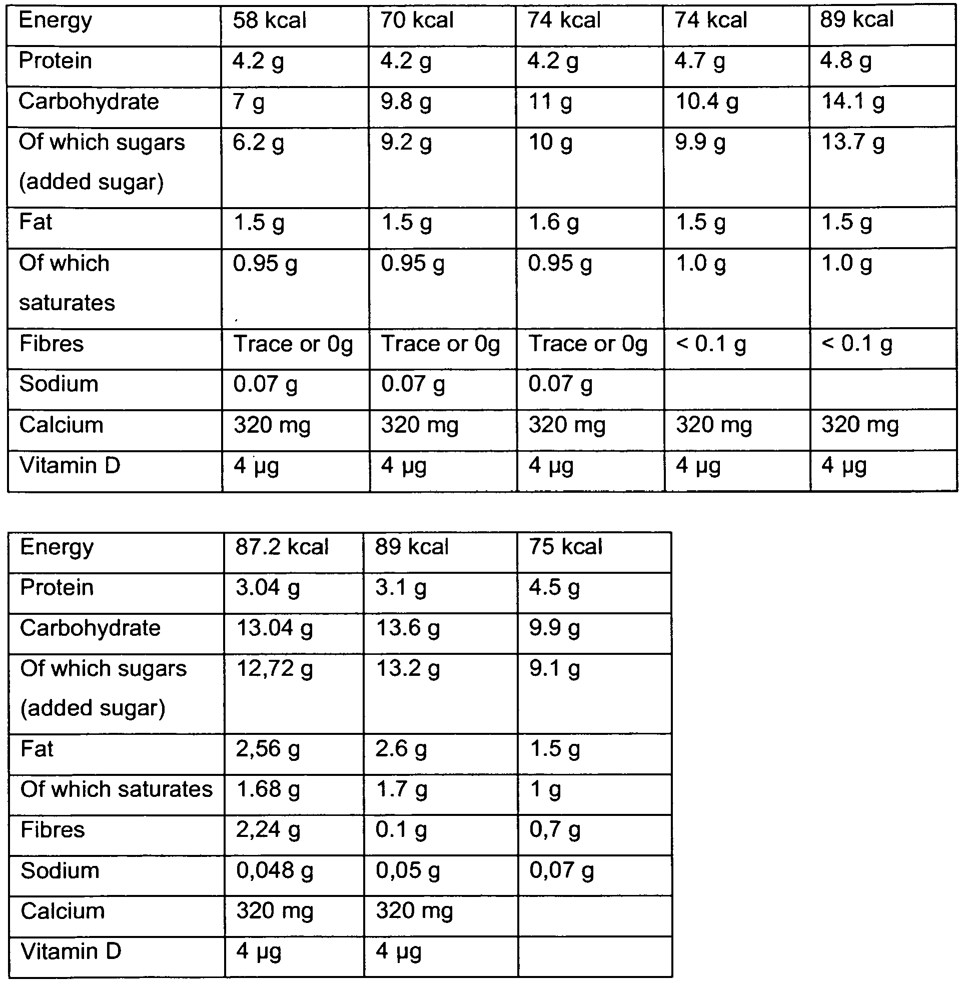

The product of the process of the invention is typically contained in a container, for example a cup, a bottle or a carton brick. The product can be thus provided in a serving amount of from higher than 30 g to 60 g, or from higher than 60 g to 90 g, or from higher than 90 g to 110 g, or from higher than 110 g to 130 g, or from higher than 130 g to 150 g, or from higher than 150 g to 200 g, of from higher than 200 g to 300 g, or from higher than 300 g to 700 g, or from higher than 700 g to 1000 g. Amounts of at most 300 g are preferred.

The absolute amounts of calcium and vitamin D depend of the serving amount and of the concentrations. In some embodiments of the invention the concentrations and the serving amount are such that the absolute amounts in a serving are as follows:
- at least 400 mg of calcium and at least 5 µg of vitamin D, or - 187.5 mg ≤ calcium < 400 mg and 5 µg ≤ vitamin D, or
- 343.75 mg ≤ calcium and 2 µg ≤ vitamin D < 5 µg, or
- 187.5 mg ≤ calcium < 343.75 mg and 3.8 µg ≤ vitamin D < 5 µg.

Similarly the concentrations and the serving amounts can be such that the absolute amounts in a serving are in ranges obtained by multiplying the limits provided above for concentrations and multiplying by 1.25.

In one embodiment the absolute amount in a serving are as follows: calcium < 1500 mg and vitamin D < 12.5 µg, preferably calcium < 1000 mg and vitamin D < 10 µg, preferably calcium < 800 mg and vitamin D < 8 µg.

The calcium can be introduced in the product in any appropriate form. For example it can be introduced in the form of tricalcium citrate.

The product of the process of the invention can comprise any ingredient typically contained in a pasteurised and fermented dairy product. These are known by the one skilled in the art. Examples include ferments, milk (animal origin), ferments, gums, sugar or syrup, pieces of fruits, nuts, fruit extracts and/or preparations thereof, flavours, aromas, further vitamins, sweeteners such as aspartame and/or acesulfam and/or steviosides, further nutrients additives, for example minerals, preservatives, stabilizers, thickeners, fibers, etc. It is mentioned that the product typically comprises water for example from 50 to 99% by weight. In one embodiment the product does not comprise vitamin K.

According to one embodiment the product further comprises a gum, for example starch, guar, xanthane, locust bean, optionally chemically depolymerised and/or chemically modified.

The product typically derives from milk, typically animal milk. The animal milk is typically cow milk, but one can use alternative animal milks such as sheep milk or goat milk. The vegetal milk can be for example soya milk. Milks, either animal or vegetal typically comprise proteins (at least 1% by weight). Animal milk for example typically comprises casein.

The product is a fermented product and thus comprises microorganisms, such as lactic acid bacteria and/or probiotics (the probiotics can be lactic acid bacteria). These are also referred to as ferments. Lactic acid bacteria are known by the one skilled in the art. Probiotics are also known by the one skilled in the art. Examples of probiotics include some Bifidobacteria and Lactobacilli, such as Bifidobacterium brevis, Lactobacillus acidophilus, lactobacillus casei, Bifidobacterium animalis, Bifidobacterium animalis lactis, Bifidobacterium infantis, bifidobacterium longum, lactobacillus casei, lactobacillus casei paracasei, lactobacillus reuteri, lactobacillus plantarum, lactobacillus rhamnosus.

In one embodiment the product is a fermented milk product or a yogurt. It is mentioned that yogurts are considered as being fermented milk products. It is believed that such forms contribute to a good intake and availability of the calcium and/or vitamin D.

Fermented animal milk products are known by the one skilled in the art. Such products are products essentially made up of animal milk, having undergone a fermentation step. The fermentation is typically done with microorganisms such as bacteria and/or yeast, preferably at least bacteria, and leads to production of fermentation products, for example lactic acid and/or to multiplication of the microorganisms. The designation "fermented milk" can depend on local legislation, but is typically given to a dairy product prepared from skimmed or full fat milk, or concentrated or powdered milk, having undergone a heat treatment at least equivalent to a pasteurization treatment, and inoculated with lactic acid producing micro-organisms such as lactobacilli (Lactobacillus acidophilus, Lb. casei, Lb. plantarum, Lb. reuteri, Lb. johnsonil), certains streptococci (Streptococcus thermophilus) bifidobacteria (Bifidobacterium bifidum, B. longum, B. breve, B. animalis) and/or lactococci (Lactococcus lactis).

Fermented vegetal milk products are known by the one skilled in the art. Such product are products essentially made up vegetal milk, having a vegetal extract as a major constituent beyond water, having undergone a fermentation step. The fermentation is typically done with microorganisms such as bacteria and/or yeast, preferably at least bacteria, and leads to production of fermentation products, for example lactic acid and/or to multiplication of the microorganisms. By vegetal extract as a major constituent, it is typically referred to a vegetal content at least equal to 50% by weight of dry matter, preferably from 70% to 100%. The vegetal milk can be for example soya milk, oat milk, rice milk, almond milk, or a mixture thereof.

According to one embodiment the product has less than 2 g fat per 100 g of product. to one embodiment the product has more than 1 g fat per 100 g of product. According to one embodiment the product has from higher than 1 to 2 g fat per 100 g of product. According to one embodiment the product is different from a cheese, for example a fresh cheese or sterilized cheese. According to one embodiment the product is different from a sterilized dessert.

The process involves pasteurizing milk (optionally introduced in the form of a powder then mixed with water), and then allowing a fermentation (after addition of ferments). After fermentation additives can be added. Calcium and vitamin D are added at that point. Calcium is typically introduced in a fruit or syrup preparation added after fermentation. Vitamin is also typically introduced in a fruit or syrup preparation added after fermentation.

It was found that it possible to accommodate quite high concentrations of both calcium, especially of calcium introduced in the form of tricalcium citrate, and vitamin D, with satisfying organoleptic properties and/or stability and/or good processing. Calcium compounds, for example tricalcium citrate, have quite low water solubility and/or a quite low water affinity. It was found surprisingly that it was possible to accommodate quite high amounts of calcium and vitamin D substantially without incompatibility. Surprisingly this was found inter-alia in fermented pasteurized products, with significant water content and/or with a quite low fat content. Such high concentrations surprisingly allow providing products with a high content of calcium and vitamin D, without increasing the food intake, and thus with allowing a balanced diet. Especially it was found that it was possible to provide balanced products with a balanced fat content, typically different from cheese products. It was found also that the high concentrations of calcium and vitamin D do not significantly impact the ferments conservation. It was found that sterilization or other thermal treatments after fermentation were not necessary. Thus it was found that it was possible to provide non cheese or not dessert milk based products high quite high loads of calcium and vitamin D.

One embodiment of the invention consists essentially in fermented pasteurised milk, based on the growth in symbiosis of strains of traditional yogurt (Lactobacillus bulgaricus and Streptococcus thermophilus) enriched with calcium and vitamin D.
Some details of possible processes to prepare the products are given below.

More specifically, a milk is typically normalized by means of mixing powdered milk with skimmed milk until obtaining a starting milk that is normalised to for example 1.85% of fat and 5.1 % of milk proteins. The starting milk is pasteurised at typically 92°C with a holding time of typically 8 minutes. The pasteurised milk is then subjected to fermenting at a temperature of about 40°C for more than 5 hours, until achieving a pH < 4.6. The fermented pasteurised milk is then typically subjected to a cooling process for example in a plate heat exchanger with water at a temperature close to 0°C as a refrigerant liquid, until the pasteurised fermented milk reaches a temperature of about 20°C, then obtaining a cool pasteurised fermented milk.

The cool fermented pasteurised milk is then typically added with an enrichment preparation comprising tricalcium citrate [for example approximately 7.1% of the enrichment preparation, comprising approximately 21% of neat calcium], vitamin D [for example approximately 5x10⁻⁵% of the enrichment preparation], optionally further ingredients such as modified starch (acetylated distarch adipate) and water.

At this point it is known that it is also possible to add artificial sweeteners and/or sugar and/or fruits and/or aromas.

This produces the end product.

Finally, the end product is packaged and, once in the package, it can be subjected to a second cooling process until the end product reaches a temperature of about 8°C, thus producing the final product.

The enrichment preparation is for example added in a sufficient amount as to provide an end product with a calcium content of approximately 3.21% and a vitamin D content of approximately 1x10⁻⁵%.

It must be taken into account that in order to determine the amount of calcium in the end product one must consider the calcium contributing both by the enrichment preparation and by the starting milk itself. This can be monitored. With an amount of calcium in the end product such as that mentioned above (for example 3.21%), at the expiry of the calcium one can achieve an amount of calcium of for example 450 mg for each 100 grams of the end product.

With respect to the vitamin D, one must take into account the losses caused by the processing of the enrichment preparation, the expiry of the enrichment preparation, the injection precision of the enrichment preparation and the expiry date of the end product. An amount of vitamin D in the end product such as that mentioned above (for example 1x10⁻⁵%) produces a minimum amount of vitamin D of about for example 4.5 µg for each 100 g of the end product.

### USE of the product

The product of the process of the invention can typically be used as a food product, more typically as functional food product.

Thus, the product of the process of the invention can be typically used by oral administration by a subject. It is mentioned that the subject can typically be a mammal, preferably a human being. The subject can typically be a female, preferably a woman.

The subject can typically be 45 years old or more, preferably a middle aged to older woman, for example of 45 years old or more. The subject can be a woman of from 45 to lower than 50, or of from 50 to lower than 55, or of from 55 to lower than 65, or of from 65 to lower than 70, or of from 70 to lower than 75, or of from 75 to lower than 80, or of from 80 to lower than 85, or of from 85 to lower than 90, or of from 96 to lower than 100. Such subjects are considered as a group (or as groups) being at risk of bone-health degradation, for example as a group (or as groups) being at risk of developing osteoporosis.

The administration can be for example of 1 or 2 servings of per day during at least 3 months days, preferably at least 6 months, preferably at least 12 months. The servings can be as provided above, preferably delivering an absolute amount of higher than or equal to 400 mg of calcium and an absolute amount of higher than or equal to 5 µg of vitamin D.

In one preferred embodiment the subject is a subject practicing "physical exercise". Herein the term physical exercise includes voluntary physical activity. This term includes activities such as physical exercise, sports, work-out, fitness practicing, training, gymnastic, musculation, low-musculation etc.

The physical activity can be of various levels, form soft to intensive. The level of physical activity can be adapted to the subject according for example to the age, fitness, and muscle function condition. Typically three different levels can be defined: a basic program, a medium program and an advanced program. It is recommended that the subject first completes the 'basic' program, and once it has successfully completed all the requirements for this level it can progress to the medium then to the advanced program. Such programs include exercises that are particularly recommended for bone health.

Indeed, the amount, density and/or architecture of the bones can be improved by mechanical loading. However, as described below, some types of exercise may be more efficient than others in increasing or maintaining bone mass. Exercises that are especially preferred to improve bone health are weight-bearing/impact exercises and strength/resistance exercises.

Weight-bearing or impact activities (such as running, jumping...) apply a greater stress on the skeleton than others exercises in weightless condition (such as cycling, rowing... ). The architecture of the skeleton is remarkably adapted to provide adequate strength and mobility to stress so that bones do not break when subjected to substantial impact, even the loads placed on bone during vigorous physical activity. Therefore, impact exercises can enhance bone formation, since mechanical loading provides an anabolic stimulus for bone. A detailed example of a suitable exercise is the following:
Initial position: Stand upright with your hand by your sides and feet slightly apart.
Execution: Jump straight up and down on the spot in a continuous fashion / Swing your arms to assist you in generating upward momentum and to assist with balance / Try and land with your knees slightly bent but minimise the knee bend / Ensure that your knees track in line with your feet during the landing.
Caution / Tips: Do not round the shoulders or flex the spine forward during the exercise / Do not land on the ground with straight legs or allow your knees to buckle inwards / The heels should not contact the ground during the exercise.

Strength or resistance activities (weight lifting) place levels of loading on bone that are beyond those seen in everyday activities. Strength or resistance exercises also increase mechanical stress on bone promoting osteogenesisis and are best for building muscle. Actions like compression, tension or torsion can generate electrical signs which stimulate bone cellular activity and mineral deposition on the stress points caused by muscle contraction. A detailed example of a suitable exercise is the following:
Initial position: Tie an elastic exercise band or rope to a stationary object / Stand about half a metre from where the band is secured with your knees slightly bent and your torso upright / Grasp the band so that your arms are almost fully extended with your shoulders slightly tilted forwards.
Execution: In a slow controlled movement, pull both arms to the sides of your body. Let the elbows guide the movement / Your shoulders should be tilted backwards as you pull the band towards your chest / Your elbows should be drawn slightly further back than your shoulders with your fists close to your chest / Hold briefly, and then return slowly to the start position and repeat.
Caution / Tips: Ensure that the band is firmly secured / Do not flex the spine forward during the exercise / Ensure that your knees are slightly bent throughout the exercise.

The training load can vary. Typically it increases with the level. Typical loads of the training are as follow:
- Exercise rate in a set: lifting phase: 2-3 seconds; lower phase; 2-3 seconds
- Number of repetitions in a set: 8 - 20 repetitions
- Number of sets: 1 - 3
- Number of various exercises: 8 - 12
- Rest time: 1-2 min between sets and various exercises

The physical exercise intensity can be form example of at least 2 METs, preferably at least 3 METs, typically from 3 to 6 METs. 3 METs can correspond to a basic level. 6 METs can correspond to an advanced level. This intensity can be delivered in set, as suggested about. This intensity and/or set can be delivered during periods of at least 15 minutes, preferably at least 30 minutes, usually at most 1 hour. The physical exercise can be for example of at least 3 METs during at least 15 minutes. The physical exercise can be for example of an energy expense of at least 100 kcal, preferably at least 200 kcal, and even at least 300 kcal for example in a time of at most one hour, for example less than 30 minutes, preferably at least 15 minutes.

It is mentioned that a MET is defined as the ratio of work metabolic rate to a standard resting metabolic rate of 1.0 (4.184 kJ) / kg. 1 MET is considered a resting metabolic rate obtained during quiet sitting. Activities have been listed as multiples of the resting MET level and range from 0.9 (sleeping) to 18 METs (running at 10.9 mph) (Ainsworth et al., 2000).

It is believed that the product of the process of the invention can contribute to providing some bone-health benefit, for example bone-loss prevention. Without intending to be bound to any theory, it is believed that combining the administration of the product and physical exercise can further improve such bone-related effects.

The bone-health can be linked to the following:
- increasing bone mineral density,
- maintaining bone density or maintaining bone mass,
- preventing or decreasing bone fragility
- preventing or decreasing bone alteration
- preventing or decreasing bone breaking occurrences
- preventing or treating osteoporosis, and/or
- preventing or decreasing bone loss occurring with aging.

Bone mass evolution is simultaneously determined by genetic, hormonal and mechanical factors. Schematically speaking, genetics define the basic structure of the skeleton, hormones regulate mineral exchanges and the mechanical forces help adapting bones to their environment exert an impact on length growth. In reality, nearly 80% of the factors involved in the formation of bone reserves are genetic. Environmental factors (nutrition, physical activity, endocrinal factors) represent a mere 20%.

Indeed, it has been shown that physical activity can contribute to bone health. Activities that are weight bearing or involve impact are most useful for maintaining bone mass. Some activities that are not weight bearing or are low impact may help improve balance and coordination and maintain muscle mass, which can help prevent falls.

Moreover, there is an important interaction between the mechanical demands link to physical activity and the availability of nutrients to manufacture bone tissue. Adequate nutrition, in particular dietary calcium intake, is also a key factor for skeletal development to ensure adequate mineralization of the structures produced in response to loading. In a meta-analysis of calcium and physical activity trials in adults, a positive response to physical activity is only achieved when calcium intake was sufficient, and likewise a response to increased calcium was only observed when physical activity was increased (Specker BL, 1996). Thus, physical activity should be encouraged for the whole population and particularly for peri and post-menopausal women that are at risk to have osteoporosis, in addition to an adequate calcium intake, because the main effects of exercise are a result of the stimulation by mechanical loading of new bone formation and the inhibition of bone loss. Without intending to be bound to any theory it is believed that the combination of physical activity and intake of calcium and vitamin D from the dairy product of the invention is especially efficient for providing bone health, especially to women subjects especially aged of at least 45 years. In this combination it is believed that there is a synergistic effect between the intake of calcium and vitamin D from the dairy product of the invention and the physical activity, or between the intake of calcium with physical activity and further intake of vitamin D from the dairy product of the invention.

The use can be for example embodied by recommending on the package and/or in commercials and/or in websites (these referring to the product) the product for women and/or for women aged of at least 45 years.

Additionally or alternatively the use can be embodied by indicating on the package and/or in commercials and/or in websites (these referring to the product), that the product can contribute to bone health as provided above. It can similarly be embodied by indication that the product can have an action for preventing, delaying, attenuating and/or treating osteoporosis.

Additionally or alternatively the use can be embodied by recommending on the package and/or in commercials and/or in websites (these referring to the product), to associate the consumption of the product with practicing physical exercise. This can be embodied for example by providing images of movements on the package, for example on the lid or cap of the package, or by developing a communication of physical activity, associated with the product: for example defining training programs to be practiced in parallel of consuming the product of the invention.

Provided below are three examples of compositions for the end product according to the embodiment of the invention disclosed above.

### Example 1 (base product)

| **Ingredient** | **Approximate contents** |
|---|---|
| Fermented pasteurised milk | 83% |
| Modified starch (acetylated distarch adipate) | 0.82% |
| Tricalcium citrate | 1.07% |
| Anhydrous citric acid | 0.06% |
| Vitamin D | 1x10⁻⁵% |
| Water | Up to |

### Example 2 (sweetened product)

| **Ingredient** | **Approximate contents** |
|---|---|
| Fermented pasteurised milk | 83% |
| Modified starch (acetylated distarch adipate) | 0.76% |
| Tricalcium citrate | 1.07% |
| Anhydrous citric acid | 0.09% |
| Vitamin D | 1x10⁻⁵% |
| Sugar (sucrose) | 3.01% |
| Acesulfame K | 0.01% |
| Aspartame | 0.01% |
| Guar flour | 0.76% |
| Water | Up to |

### Example 3 (strawberry product)

| **Ingredient** | **Approximate contents** |
|---|---|
| Fermented pasteurised milk | 83% |
| Modified starch (acetylated distarch adipate) | 0.42% |
| Tricalcium citrate | 1.07% |
| Anhydrous citric acid | 0.04% |
| Vitamin D | 1x10⁻⁵% |
| Sugar (sucrose) | 3.70% |
| Acesulfame K | 0.01% |
| Aspartame | 0.01% |
| Guar flour | 0.04% |
| Strawberry | 5.50% |
| 4% carminic acid | 0.06% |
| Water | Up to |

### Example 4:

The products of examples 1 to 3 are conditioned in a 125 g yogurt cup.

### Example 5:

The products of example 4 are administered orally to a group of 20 women aged of at least 45 years, in a amount of one 125 g yogurt cup, daily during more than 6 months.
Good bone health is promoted.

### Example 6

The products of example 4 are administered orally to a group of 20 women aged of at least 45 years practicing physical exercise at least 3 time a week, in a amount of one 125 yogurt cup, daily during more than 6 months.
Good bone health is promoted.

Naturally, since the principles of the invention remain the same, the details of the embodiment can vary a lot with respect to those described and illustrated here as nonlimiting examples, without exceeding the scope of protection defined by the following claims.

## Claims

1. A process for preparing a pasteurised and fermented dairy product, involving pasteurising milk, said milk being optionally introduced in the form of a powder then mixed with water, then allowing a fermentation after addition of ferments, and adding calcium and vitamin D additives after fermentation, **characterised in that** the pasteurized and fermented dairy product includes the following amounts of calcium and vitamin D, for each 100 g of the product:
- at least 320 mg of calcium and at least 4 µg of vitamin D, or
- 150 mg ≤ calcium < 320 mg and 4 µg ≤ vitamin D < 10 µg.

2. A process according to any of the preceding claims, wherein calcium is introduced in the form of tricalcium citrate.

3. A process according to any to the preceding claims, further comprising a gum.

4. A process according to any of the preceding claims, being a yogurt or a fermented milk product.

5. A process according to any of the preceding claims, wherein the amounts are as follows:
- calcium = 320 mg and vitamin D = 4 µg, or
- 320 mg < calcium < 1000 mg and 4 µg < vitamin D < 10 µg.

6. A process according to any of the preceding claims, contained in a container, in a serving amount of from higher than 30 g to 60 g, or from higher than 60 g to 90 g, or from higher than 90 g to 110 g, or from higher than 110 g to 130 g, or from higher than 130 g to 150 g, or from higher than 150 g to 200 g, of from higher than 200 g to 300 g, or from higher than 300 g to 700 g, or from higher than 700 g to 1000 g.

7. A process according to any of the preceding claims, **characterised in that** the freshly made pasteurised and fermented dairy product includes a calcium content of approximately 3.21 % and a vitamin D content of approximately 1x10⁻⁵%.

8. A process according to any of the preceding claims, **characterised in that** the pasteurised and fermented dairy product includes an amount of calcium of about 450 mg at calcium expiry and an amount of vitamin D of about 4.5 µg at the expiry date of the product for each 100 g of the product.

9. A process according to any of the claims 1 to 7, **characterised in that** the freshly made pasteurised and fermented dairy product indudes:
| **Ingredient** | **contents** |
|---|---|
| Fermented pasteurised milk | 83% |
| Modified starch (acetylated distarch adipate) | 0,82% |
| Tricalcium citrate | 1.07% |
| Anhydrous citric acid | 0.06% |
| Vitamin D | 1x10⁻⁵% |
| Water | Up to |

10. A process according to any of claims 1 to 7, **characterised in that** the freshly made pasteurised and fermented dairy product includes:
| **Ingredient** | **contents** |
|---|---|
| Fermented pasteurised milk | 83% |
| Modified starch (acetylated distarch adipate) | 0.76% |
| Tricalcium citrate | 1.07% |
| Anhydrous citric add | 0.09% |
| Vitamin D | 1x10⁻⁵%. |
| Sugar (sucrose) | 3.01% |
| Acesulfame K | 0.01% |
| Aspartame | 0.01% |
| Guar flour | 0.76% |
| Water | Up to |

11. A process according to any of claims 1 to 7, **characterised In that** the freshly made pasteurised and fermented dairy product Includes:
| **Ingredient** | **contents** |
|---|---|
| Fermented pasteurised milk | 83% |
| Modified starch (acetylated distarch adipate) | 0.42% |
| Tricalcium citrate | 1.07% |
| Anhydrous citric acid | 0.04% |
| Vitamin D | 1x10⁻⁵% |
| Sugar (sucrose) | 3.70% |
| Acesulfame K | 0.01% |
| Aspartame | 0.01% |
| Guar flour | 0.04% |
| Strawberry | 5.50% |
| 4% carminic acid | 0.06% |
| Water | Up to |

12. A process according to any of the preceding claims, wherein the product is different from products having the following nutritional profiles, per 100 g of product

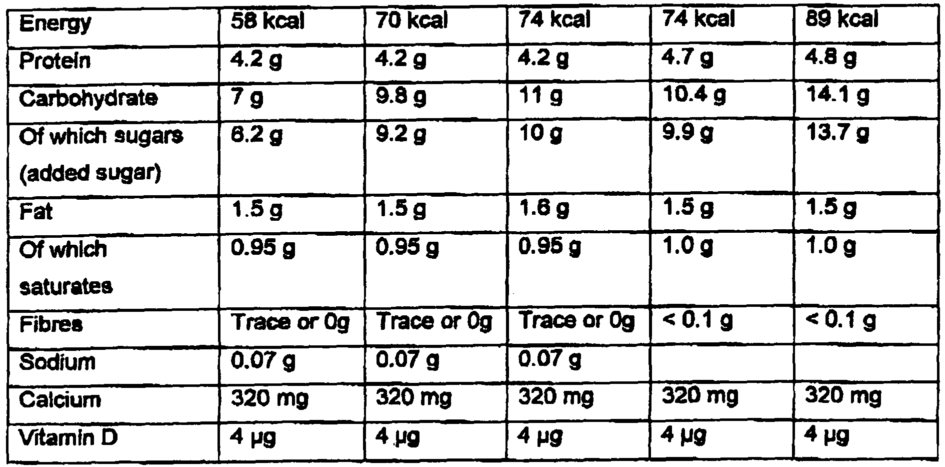

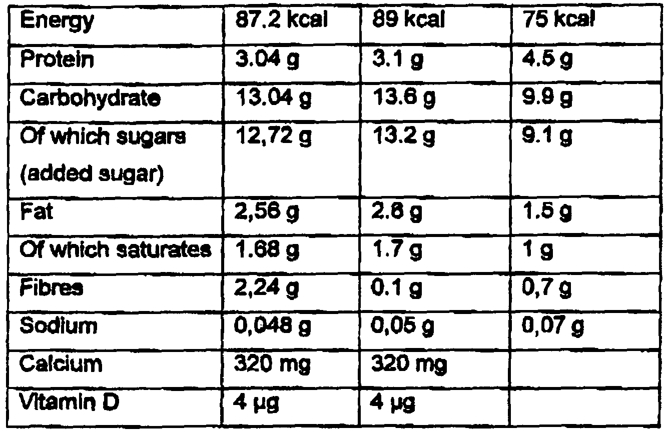

13. A process according to any of the preceding claims, wherein the product has less than 2 g fat per 100 g of the product.

14. A process according to any of the previous claims, **characterised in that**:
- a fresh milk is normalized by means of mixing powdered milk with skimmed milk until obtaining a starting milk that is normalised to 1.85% of fat and 5.1% of milk proteins;
- the starting milk is pasteurised at 92°C with a holding time of 8 minutes;
- the pasteurised milk is then subjected to fermenting at a temperature of about 40°C for more than 5 hours, until achieving a pH < 4.6;
- the fermented pasteurised milk is subjected to a cooling process In a plate heat exchanger with water at a temperature close to 0°C as a refrigerant liquid, until the pasteurised fermented milk reaches a temperature of about 20°C, thus obtaining a cool pasteurised fermented milk;
- the cool fermented pasteurised milk is added an enrichment preparation comprising tricalcium citrate, vitamin D , modified starch and water;
- optionally, artificial sweeteners and/or sugar and/or fruit and/or aromas are added to the cool enriched fermented pasteurised milk, thus obtaining the end product;
- the end product is packaged and, once in the package, it Is subjected to a second cooling process until the end product reaches a temperature of about 8°C, thus producing the final product.

15. A process according to claim 14, **characterised in that** the enrichment preparation is added in a sufficient amount as to provide an end product with a calcium content of approximately 3:21% and a vitamin D content of approximately 1x10⁻⁵%.

## Patentansprüche

1. Verfahren zur Herstellung eines pasteurisierten und fermentierten Milchprodukts, einschließlich der Pasteurisierung, wobei man die Milch als Option in Form von Milchpulver hinzufügen und mit Wasser mischen kann und anschließend zur Fermentierung die Fermente sowie nach diesem Vorgang Calcium und Vitamin D hinzugibt, **dadurch gekennzeichnet, dass** das pasteurisierte und fermentierte Milchprodukt pro 100 g die folgenden Mengen an Calcium und Vitamin D enthält:
- mindestens 320 mg Calcium und mindestens 4 µg Vitamin D oder
- 150 mg ≤ Calciummenge < 320 mg und 4 µg ≤ Vitamin-D-Menge < 10 µg.

2. Verfahren nach Anspruch 1, wobei man das Calcium als Calciumcitrat hinzugibt.

3. Verfahren nach einem der vorherigen Ansprüche, das außerdem die Hinzugabe von Xanthan umfasst.

4. Verfahren nach einem der vorherigen Ansprüche, wobei es sich um ein Joghurt oder ein fermentiertes Milchprodukt handelt.

5. Verfahren nach einem der vorherigen Ansprüche mit folgenden Stoffmengen
- 320 mg Calcium und 4 µg Vitamin D oder
- 320 mg < Calciummenge < 1000 mg und 4 µg < Vitamin-D-Menge < 10 µg.

6. Verfahren nach einem der vorherigen Ansprüche, wobei das Produkt in folgenden Serviermengen in einen Behälter abgefüllt wird: von über 30 g bis 60 g, von über 60 g bis 90 g, von über 90 g bis 110 g, von über 110 g bis 130 g, von über 130 g bis 150 g, von über 150 g bis 200 g, von über 200 g bis 300 g, von über 300 g bis 700 g oder von über 700 g bis 1000 g.

7. Verfahren nach einem der vorherigen Ansprüche, **dadurch gekennzeichnet, dass** das frisch hergestellte pasteurisierte und fermentierte Milchprodukt einen Calciumgehalt von etwa 3,21% und einen Vitamin-D-Gehalt von etwa 10⁻⁵% aufweist.

8. Verfahren nach einem der vorherigen Ansprüche, **dadurch gekennzeichnet, dass** das pasteurisierte und fermentierte Milchprodukt pro 100 g zum Verfalldatum des Calciums etwa 450 mg Calcium und zum Verfalldatum des Produkts etwa 4,5 µg Vitamin D enthält.

9. Verfahren nach einem der vorherigen Ansprüche 1 bis 7, **dadurch gekennzeichnet, dass** das frisch hergestellte pasteurisierte und fermentierte Milchprodukt folgende Stoffmengen enthält:
| **Inhaltsstoff** | **Anteil** |
|---|---|
| Fermentierte pasteurisierte Milch | 83% |
| Modifizierte Stärke (acetyliertes Distärkeadipat) | 0,82% |
| Tricalciumcitrat | 1,07% |
| Zitronensäure-Anhydrid | 0,06% |
| Vitamin D | 10⁻⁵% |
| Wasser | Rest (bis 100%) |

10. Verfahren nach einem der vorherigen Ansprüche 1 bis 7, **dadurch gekennzeichnet, dass** das frisch hergestellte pasteurisierte und fermentierte Milchprodukt folgende Stoffmengen enthält:
| **Inhaltsstoff** | **Anteil** |
|---|---|
| Fermentierte pasteurisierte Milch | 83% |
| Modifizierte Stärke (acetyliertes Distärkeadipat) | 0,76% |
| Tricalciumcitrat | 1,07% |
| Zitronensäure-Anhydrid | 0,09% |
| Vitamin D | 10⁻⁵% |
| Zucker (Saccharose) | 3,01% |
| Aspartam | 0,01% |
| Guarmehl | 0,76% |
| Wasser | Rest (bis 100%) |

11. Verfahren nach einem der vorherigen Ansprüche 1 bis 7, **dadurch gekennzeichnet, dass** das frisch hergestellte pasteurisierte und fermentierte Milchprodukt folgende Stoffmengen enthält:
| **Inhaltsstoff** | **Anteil** |
|---|---|
| Fermentierte pasteurisierte Milch | 83% |
| Modifizierte Stärke (acetyliertes Distärkeadipat) | 0,42% |
| Tricalciumcitrat | 1,07% |
| Zitronensäure-Anhydrid | 0,04% |
| Vitamin D | 10⁻⁵% |
| Zucker (Saccharose) | 3,70% |
| Acesulfam K | 0,01% |
| Aspartam | 0,01% |
| Guarmehl | 0,04% |
| Erdbeere | 5,50% |
| 4%-ige Karminsäure | 0,06% |
| Wasser | Rest (bis 100%) |

12. Verfahren nach einem der vorherigen Ansprüche, wobei sich die einzelnen Produktvarianten durch die folgenden Nährstoffgehalte pro 100 g unterscheiden:
| Energie | 58 kcal | 70 kcal | 74 kcal | 74 kcal | 89 kcal | 87,2 kcal | 89 kcal | 75 kcal |
|---|---|---|---|---|---|---|---|---|
| Protein | 4,2 g | 4,2 g | 4,2 g | 4,7 g | 4,8 g | 3,04 g | 3,1 g | 4,5 g |
| Kohlenhydrate | 7g | 9,8 g | 11 g | 10,4 g | 14,1 g | 13,04 g | 13,6 g | 9,9 g |
| Davon Zucker (Zuckerzusatz) | 8,2 g | 9,2 g | 10g | 9,9 g | 13,7 g | 12,72 g | 13,2 g | 9,1 g |
| Fette | 1,5 g | 1,5 g | 1,6 g | 1,5 g | 1,5 g | 2,56 g | 2,6 g | 1,5 g |
| Davon gesättigte Fette | 0,95 g | 0,95 g | 0,95 g | 1,0 g | 1,0 g | 1,68 g | 1,7 g | 1 g |
| Faserstoffe | Spuren oder 0 g | Spuren oder 0 g | Spuren oder 0 g | < 0,1 g | < 0,1 g | 2,24 g | 0,1 g | 0,7 g |
| Natrium | 0,07 g | 0,07 g | 0,07 g | | | 0,048 g | 0,05 g | 0,07 g |
| Calcium | 320 mg | 320 mg | 320 mg | 320 mg | 320 mg | 320 mg | 320 mg | |
| Vitamin D | 4 µg | 4 µg | 4 µg | 4 µg | 4 µg | 4 µg | 4 µg | |

13. verfahren nach einem der vorherigen Ansprüche, wobei das Produkt pro 100 g weniger als 2 g Fett enthält.

14. verfahren nach einem der vorherigen Ansprüche, das sich durch den folgenden Ablauf auszeichnet:
- Nach der Mischung von Milchpulver und entrahmter Milch erhält man eine Ausgangsmilch mit 1,85% Fett und 5,1% Milchproteinen als Standardwerte;
- diese Ausgangsmilch wird acht Minuten lang bei einer Temperatur von 92ºC pasteurisiert;
- diese pasteurisierte Milch fermentiert anschließend bei einer Temperatur von etwa 40ºC über fünf Stunden lang, bis sich ein pH-Wert unter 4,6 einstellt;
- die derart fermentierte pasteurisierte Milch kühlt sich in einem Plattenwärmetauscher mit Wasser bei annähernd 0ºC als Kühlmittel auf eine Temperatur von etwa 20ºC ab;
- zur nun abgekühlten, fermentierten und pasteurisierten Milch fügt man eine anreichernde Zubereitung aus Calciumcitrat, Vitamin D, modifizierter Stärke und Wasser;
- als Optionen kann man zur angereicherten, abgekühlten, fermentierten und pasteurisierten Milch künstliche Süßstoffe, Zucker, Früchte und/oder Aromastoffe hinzufügen und erhält so das Endprodukt;
- dieses Endprodukt wird verpackt und nachfolgend in der Packung in einem zweiten Kühlungsprozess auf eine Endtemperatur von etwa 8ºC abgekühlt.

15. verfahren nach Anspruch 14, **dadurch gekennzeichnet, dass** man die anreichernde Zubereitung in einer hinreichenden Menge hinzufügt, sodass das Endprodukt einen Calciumgehalt von etwa 3,21% und einen und einen Vitamin-D-Gehalt von etwa 10⁻⁵% aufweist.

## Revendications

1. Procédé de préparation d'un produit laitier pasteurisé et fermenté, comprenant une pasteurisation d'un lait, ledit lait étant éventuellement introduit sous la forme d'une poudre, puis mélangé avec de l'eau, puis une fermentation après l'ajout de ferments, et l'ajout d'additifs calcium et vitamine D après la fermentation, **caractérisé en ce que** le produit laitier pasteurisé et fermenté comprend les quantités suivantes de calcium et de vitamine D, pour 100 g du produit :
- au moins 320 mg de calcium et au moins 4 µg de vitamine D ou
- 150 mg ≤ calcium ≤ 320 mg et 4 µg ≤ vitamine D ≤ 10 µg.

2. Procédé selon l'une quelconque des revendications précédentes, dans lequel le calcium est introduit sous la forme de citrate de tricalcium.

3. Procédé selon l'une quelconque des revendications précédentes, comprenant également une gomme.

4. Procédé selon l'une quelconque des revendications précédentes, qui est un yaourt ou un produit à base de lait fermenté.

5. Procédé selon l'une quelconque des revendications précédentes, dans lequel les quantités sont les suivantes :
- calcium = 320 mg et vitamine D = 4 µg ou
- 320 mg < calcium < 1 000 mg et 4 µg < vitamine D < 10 µg.

6. Procédé selon l'une quelconque des revendications précédentes, contenu dans un contenant, en une quantité de service de plus de 30 g à 60 g, ou de plus de 60 g à 90 g, ou de plus de 90 g à 110 g, ou de plus de 110 g à 130 g, ou de plus de 130 g à 150 g, ou de plus de 150 g à 200 g, ou de plus de 200 g à 300 g, ou de plus de 300 g à 700 g, ou de plus de 700 g à 1 000 g.

7. Procédé selon l'une quelconque des revendications précédentes, **caractérisé en ce que** le produit laitier pasteurisé et fermenté fraîchement préparé comprend une teneur en calcium d'approximativement 3,21 % et une teneur en vitamine D d'approximativement 1 x 10⁻⁵ %.

8. Procédé selon l'une quelconque des revendications précédentes, **caractérisé en ce que** le produit laitier pasteurisé et fermenté comprend une quantité de calcium d'environ 450 mg à la date d'expiration du calcium et une quantité de vitamine D d'environ 4,5 µg à la date d'expiration du produit pour 100 g du produit.

9. Procédé selon l'une quelconque des revendications 1 à 7, **caractérisé en ce que** le produit laitier pasteurisé et fermenté fraîchement préparé comprend :
| Ingrédient | Teneur |
|---|---|
| Lait pasteurisé fermenté | 83 % |
| Amidon modifié (adipate de diamidon acétylé) | 0,82 % |
| Citrate de tricalcium | 1,07 % |
| Acide citrique anhydre | 0,08 % |
| Vitamine D | 1 x 10⁻⁵ % |
| Eau | Jusqu'à |

10. Procédé selon l'une quelconque des revendications 1 à 7, **caractérisé en ce que** le produit laitier pasteurisé et fermenté fraîchement préparé comprend :
| Ingrédient | Teneur |
|---|---|
| Lait pasteurisé fermenté | 83 % |
| Amidon modifié (adipate de diamidon acétylé) | 0,76 % |
| Citrate de tricalcium | 1,07 % |
| Acide citrique anhydre | 0,09 % |
| Vitamine D | 1 x 10⁻⁵ % |
| Sucre (sucrose) | 3,01 % |
| Acésulfame K | 0,01 % |
| Aspartame | 0,01 % |
| Farine de guar | 0,76 % |
| Eau | Jusqu'à |

11. Procédé selon l'une quelconque des revendications 1 à 7, **caractérisé en ce que** le produit laitier pasteurisé et fermenté fraîchement préparé comprend :
| Ingrédient | Teneur |
|---|---|
| Lait pasteurisé fermenté | 83 % |
| Amidon modifié (adipate de diamidon acétylé) | 0,42 % |
| Citrate de tricalcium | 1,07 % |
| Acide citrique anhydre | 0,04 % |
| Vitamine D | 1 x 10⁻⁵ % |
| Sucre (sucrose) | 3,70 % |
| Acésulfame K | 0,01 % |
| Aspartame | 0,01 % |
| Farine de guar | 0,04 % |
| Fraise | 5,50 % |
| Acide carminique 4 % | 0,06 % |
| Eau | Jusqu'à |

12. Procédé selon l'une quelconque des revendications précédentes, dans lequel le produit est différent des produits ayant les profils nutritionnels suivants, pour 100 g de produit :

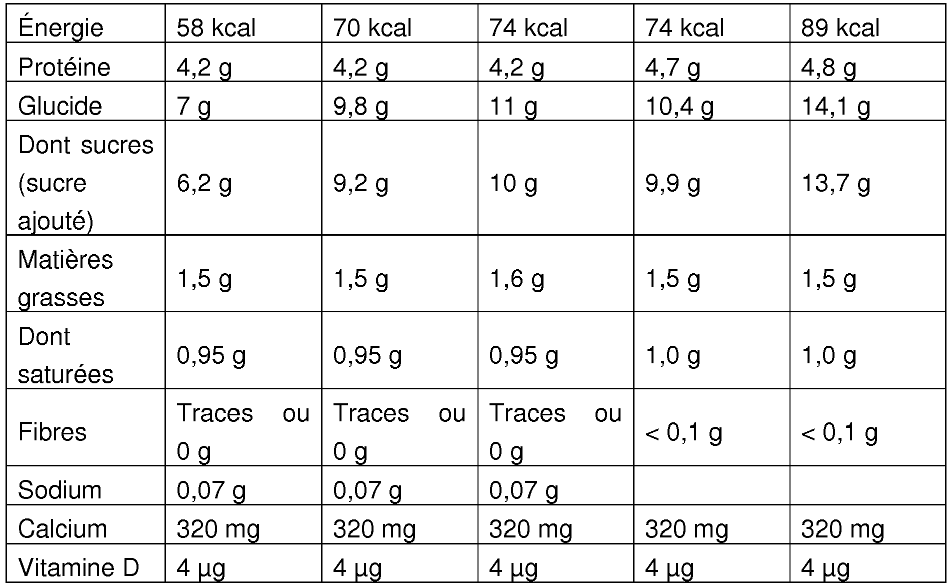

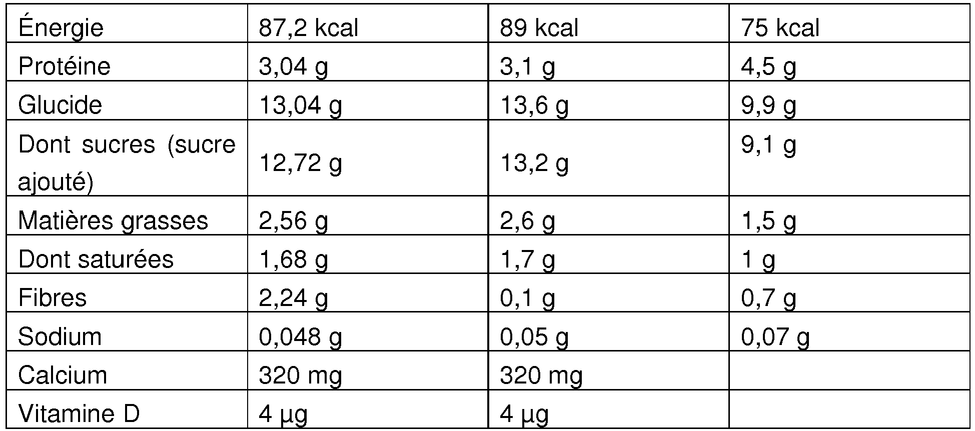

13. Procédé selon l'une quelconque des revendications précédentes, dans lequel le produit contient moins de 2 g de matières grasses pour 100 g du produit.

14. Procédé selon l'une quelconque des revendications précédentes, **caractérisé en ce que** :
- un lait frais est normalisé par mélange de lait en poudre avec du lait écrémé jusqu'à l'obtention d'un lait de départ qui est normalisé à 1,85 % de matières grasses et 5,1 % de protéines de lait ;
- le lait de départ est pasteurisé à 92 °C avec un temps de maintien de 8 minutes ;
- le lait pasteurisé est ensuite soumis à une fermentation à une température d'environ 40 °C pendant plus de 5 heures, jusqu'à atteindre un pH < 4,6 ;
- le lait pasteurisé fermenté est soumis à un procédé de refroidissement dans un échangeur de chaleur à plaques avec de l'eau à une température proche de 0 °C en tant que liquide réfrigérant, jusqu'à ce que le lait fermenté pasteurisé atteigne une température d'environ 20 °C afin d'obtenir un lait fermenté pasteurisé froid ;
- une préparation d'enrichissement comprenant du citrate de tricalcium, de la vitamine D, de l'amidon modifié et de l'eau est ajoutée au lait pasteurisé fermenté froid ;
- des édulcorants artificiels et/ou du sucre et/ou des fruits et/ou des arômes sont éventuellement ajoutés au lait pasteurisé fermenté enrichi froid afin d'obtenir le produit fini ;
- le produit fini est emballé et, une fois dans l'emballage, il est soumis à un second procédé de refroidissement jusqu'à ce que le produit fini atteigne une température d'environ 8 °C afin d'obtenir le produit final.

15. Procédé selon la revendication 14, **caractérisé en ce que** la préparation d'enrichissement est ajoutée en une quantité suffisante pour obtenir un produit fini ayant une teneur en calcium d'approximativement 3,21 % et une teneur en vitamine D d'approximativement 1 x 10⁻⁵ %.
